Europäisches Patentamt

European Patent Office

Office européen des brevets

(19)

(11) EP 0 718 378 B1

(12) EUROPÄISCHE PATENTSCHRIFT

(45) Veröffentlichungstag und Bekanntmachung des Hinweises auf die Patenterteilung:
07.11.2001 Patentblatt 2001/45

(51) Int Cl.⁷: **C09C 1/00**, C09C 3/06, C08K 9/02, C09D 7/12, A61K 7/00, C09C 1/40

(21) Anmeldenummer: 95119465.3

(22) Anmeldetag: 11.12.1995

(54) **Nicht glänzende Farbpigmente**

Non-brilliant coloured pigments

Pigments colorés non brillants

(84) Benannte Vertragsstaaten:
**DE ES FR GB IT**

(30) Priorität: 21.12.1994 DE 4445394

(43) Veröffentlichungstag der Anmeldung:
26.06.1996 Patentblatt 1996/26

(73) Patentinhaber: **MERCK PATENT GmbH**
**64293 Darmstadt (DE)**

(72) Erfinder:
• **Schmidt, Christoph, Dr.**
**D-65830 Kriftel (DE)**

• **Seibel, Claudia**
**D-64853 Otzberg (DE)**

(56) Entgegenhaltungen:
EP-A- 0 077 959          EP-A- 0 220 617
EP-A- 0 562 329          WO-A-84/01909
DE-A- 2 313 331          DE-A- 4 441 223
US-A- 4 499 143

• **DATABASE WPI Week 8830 Derwent Publications Ltd., London, GB; AN 88-209193 XP002006468 & JP-A-63 145 410 (HOSOKAWA MICRON) , 17.Juni 1988**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft nicht glänzende eisenoxidhaltige Farbpigmente auf Basis von trocken gemahlenen anorganischen plättchenförmigen Trägern der Teilchengröße < 25 μm, die sich dadurch auszeichnen, daß es sich bei der Eisenoxidbeschichtung um gelbes $FeO(OH)$ und/oder $Fe_2O_3$ handelt.

Klassische Absorptionspigmente, wie z.B. Eisenoxide, bestehen üblicherweise aus sehr kleinen Partikeln mit einer mittleren Teilchengröße von typischerweise 0,1-1 μm, die bestimmte Wellenlängen bzw. Wellenlängenbereiche des einfallendes Lichtes absorbieren und das restliche Licht im wesentlichen diffus reflektieren und somit die Komplementärfarbe des absorbierten Lichtes erzeugen. Derartige Pigmente weisen im allgemeinen eine rauhe, unregelmäßige Oberfläche auf und sind mehr oder weniger stark opak. Die optischen Eigenschaften werden durch die mittlere Größe der Pigmentpartikel beeinflußt, wobei die Farbreinheit und Farbtiefe mit abnehmender mittlerer Partikelgröße zunimmt. Besonders günstige Eigenschaften weisen Pigmentpartikel mit einem mittleren Durchmesser von weniger als 1 μm auf. Derartige ultrafeine Pigmentpartikel zeichnen sich aber im allgemeinen durch eine schlechte Dispergierbarkeit aus, welche mit abnehmender Partikelgröße zunimmt.

Bei den Absorptionspigmenten haben insbesondere Eisenoxidpigmente eine große wirtschaftliche Bedeutung, vor allem aufgrund ihrer Wetterbeständigkeit, Lichtechtheit und der breiten Farbskala, die von Gelb über Rot und Braun bis Schwarz reicht. Farbige eisenoxidhaltige plättchenförmige Pigmente, werden in der Literatur vielfach beschrieben. Insbesondere werden dabei $Fe_2O_3$-Schichten, gegebenenfalls mit anderen Metalloxiden, wie z.B. $TiO_2$, auf einen plättchenförmigen anorganischen Träger, wie z.B. Glimmer aufgebracht. Derartige Pigmente sind aus der DE 23 13 331 bekannt. In der DE 41 37 764 werden Eisenoxidpigmente auf Basis von $SiO_2$, $TiO_2$ oder $ZrO_2$-Plättchen beansprucht. In Cosmetics & Toiletries, Vol. 107, No. 4, S. 53 ff. werden transparente eisenoxidhaltige Pigmente beschrieben, die durch Beschichtung von $FeO(OH)$ oder $Fe_2O_3$ auf Glimmer erhalten werden.

Eisenoxidhaltige Pigmente auf Basis plättchenförmiger Substrate, die sogenannten Perlglanzpigmente, weisen aufgrund der glatten Oberfläche und des größeren Partikeldurchmessers, typischerweise bei 15-100 μm, andere optische Eigenschaften auf als Absorptionspigmente. Perlglanzpigmente zeichnen sich durch einen hohen Glanz und ein geringes Deckvermögen aus - Eigenschaften, die auf das transparente oder semitransparente Substratmaterial zurückgeführt werden müssen. Für viele Anwendungen werden aber gutdeckende Farbpigmente ohne Glanz benötigt, z.B. in kosmetischen Formulierungen wie Pudern.

Es bestand daher die Aufgabe glanzlose Farbpigmente zu finden, die neben der Körperfarbe ein hohes Deckvermögen aufweisen, gut dispergierbar sind und keinen der oben genannten Nachteile aufweisen.

Überraschenderweise wurde nun gefunden, daß man nicht glänzende eisenoxidhaltige Pigmente mit hoher Farbstärke erhält, wenn man trocken gemahlene anorganische plättchenförmige Träger der Teilchengröße < 25 μm mit gelbem $FeO(OH)$ und/oder $Fe_2O_3$ beschichtet.

Aus der EP 0 659 843 A1, einem Stand der Technik nach A 54(3), (4) EPÜ, sind Eisenoxidpigmente auf Basis eines unregelmäßig geformten Glimmerteilchens bekannt. Das dort beschriebene Pigment wird nach einem Verfahren hergestellt das zu roten Eisenoxidpigmenten führt.

Die erfindungsgemäßen Farbpigmente zeigen in Bezug auf Farbstärke, Farbnuancierung, Glanz und Deckvermögen ganz wesentliche Unterschiede im Vergleich zu den eisenoxidhaltigen Perlglanzpigmenten. Das zeigt eindeutig, daß nicht allein die chemische Zusammensetzung der Beschichtung für die Eigenschaften des Pigments verantwortlich sind, sondern, daß eine ganze Reihe weiterer Faktoren eine Rolle spielen, insbesondere der Aufbau und die Beschaffenheit des Basissubstrats als auch das Beschichtungsverfahren. Durch die Verwendung eines glanzlosen Basissubstrats mit unregelmäßiger Teilchenform in Kombination mit $FeO(OH)$- bzw. $Fe_2O_3$-Belegungen auf der Substratoberfläche erhält man Farbpigmente, die aufgrund der irregulären Form der Oberfläche und der Lichtabsorption nur eine Farbe zeigen und keinen Glanz besitzen.

Gegenstand der Erfindung sind daher nicht glänzende eisenoxidhaltige Farbpigmente auf Basis von trocken gemahlenen anorganischen plättchenförmigen Trägern der Teilchengröße < 25 μm, dadurch gekennzeichnet, daß glanzlose Basissubstrate mit unregelmäßiger Teilchenform mit gelbem $FeO(OH)$ und/oder $Fe_2O_3$ beschichtet werden.

Gegenstand der Erfindung ist weiterhin ein Verfahren zur Herstellung der erfindungsgemäßen Pigmente, das sich dadurch auszeichnet, daß man eine wäßrige Suspension des trocken gemahlenen anorganischen plättchenförmigen Trägers vorlegt, bei Temperaturen von 5-40 °C und einem pH-Wert von 6-9 eine wäßrige Eisen(II)salzlösung unter Rühren zugesetzt und durch die Reaktionslösung Luft und/oder reinen Sauerstoff leitet, danach das beschichtete Pigment abtrennt, wäscht, trocknet und gegebenenfalls bei Temperaturen von 400-1000 °C glüht.

Wesentlicher Bestandteil des erfindungsgemäßen Farbpigments ist das Basissubstrat. Als plättchenförmiger anorganischer Träger kommen alle Schichtsilikate, wie z.B. Glimmer, Talk, Kaolin oder Sericit, aber auch synthetischer Glimmer in Frage. Bevorzugt wird Glimmer, wie z.B. Muskovit oder Phlogopit, eingesetzt. In der Regel werden Substrate mit einer Teilchengröße < 25 μm, und insbesondere < 15 μm, eingesetzt. Die als Substrate verwendeten Ausgangsmaterialien sind bekannt und lassen sich durch trockenes Vermahlen der plättchenförmigen Substrate und nachfolgendes Klassieren in der gewünschten Größenordnung gewinnen. Die trocken gemahlenen Schichtsilikate haben ihre ursprüngliche Plättchenform weitestgehend verloren. So erhält man beispielsweise bei der trockenen Mahlung von Muskovit-Glimmer in einer Kugelmühle ein weißes glanzloses Pulver, welches im wesentlichen aus unregelmäßig geformten Partikeln ohne Plättchenstruktur besteht.

Das erfindungsgemäße Verfahren ist einfach und leicht zu handhaben. Die Beschichtung erfolgt naßchemisch, wobei zunächst die trocken gemahlenen Schichtsilikate in Wasser suspendiert werden. Anschließend erfolgt die Zugabe eines Eisen(II)salzes, wie z.B. Ammonium-Eisen(II)sulfat, Eisen(II)halogenid oder insbesondere Eisen(II)sulfat, bei Temperaturen im Bereich von 5 bis 40 °C, vorzugsweise 10-30 °C, wobei der pH-Wert des Reaktionsgemisches in einem Bereich gehalten wird, der die Metallsalzhydrolyse bewirkt. Am zweckmäßigsten geschieht dies durch gleichzeitige Zugabe einer Base, wie z.B. NaOH, KOH, $Na_2CO_3$ oder Ammoniak. Es ist jedoch auch möglich ein geeignetes Puffersystem zu verwenden. Der pH-Wert sollte vorzugsweise oberhalb von 6 liegen, insbesondere bei 6-9.

Während und/oder nach der Zugabe des Eisen(II)salzes, vorzugsweise als wäßrige Eisen(II)salz-Lösung, wird als Oxidationsmittel Luft und/oder reiner Sauerstoff durch die Reaktionslösung geleitet, wobei sich das Eisenoxid als Eisenoxidhydrat auf dem Substrat derart niederschlägt, daß es zu keinen Nebenfällungen in der Suspension kommt.

Das fertige Farbpigment wird zuletzt aus dem Reaktionsgemisch abgetrennt, mit Wasser gewaschen und in der Regel bei Temperaturen von 80-120 °C getrocknet. Man erhält ein Gelbpigment, das sich beim Entwässern bei Temperaturen von 400-1000 °C orange verfärbt aufgrund der Umwandlung des FeO(OH) in das $Fe_2O_3$.

Diese eisenoxidhaltige Schicht kann je nach gewünschter Farbstärke Dicken bis zu etwa 300 nm, vorzugsweise 10-80 nm aufweisen. Die erfindungsgemäßen Farbpigmente können bis etwa 80 Gew.%, vorzugsweise bis 50 Gew.%, an FeO(OH) bzw. $Fe_2O_3$ enthalten.

Nach dem erfindungsgemäßen Verfahren können alle bekannten Substrate - sowohl plättchenförmige als auch nicht plättchenförmige Substrate mit einer Teilchengröße von 1-25 µm mit einer FeO(OH) oder $Fe_2O_3$-Schicht beschichtet werden.

Geeignete Substrate für das Verfahren sind insbesondere Schichtsilikate, wie z.B. Talk, Kaolin, Sericit oder Glimmer, plättchenförmiges Wismutoxichlorid, Bariumsulfat, synthetischer Glimmer, Metalleffektpigmente, wie plättchenförmiges Eisenoxid, Aluminiumplättchen, z.B. Standard von der Fa. Eckert, Effektpigmente, wie z.B. Paliochrom® von der BASF, sowie Perlglanzpigmente - mit Metalloxiden beschichtete Glimmerschuppenpigmente der Fa. E. Merck, Darmstadt unter dem Handelsnamen Iriodin. Letztere sind z.B. aus den deutschen Patenten und Patentanmeldungen 14 67 468, 19 59 998, 20 09 566, 22 14 545, 22 15 191, 22 44 298, 23 13 331, 25 22 272, 31 37 808, 31 37 809, 31 51 343, 31 51 354, 31 51 355, 32 11 602 und 32 35 017 bekannt. Besonders bevorzugt werden mit $TiO_2$ und/oder $Fe_2O_3$ beschichtete Glimmerpigmente sowie unbeschichtete oder mit ein oder mehreren Metalloxiden beschichtete $SiO_2$-Flakes, Glasflakes, Keramikflakes oder synthetische trägerfreie Plättchen eingesetzt.

In manchen Fällen ist es zweckmäßig die erfindungsgemäßen Farbpigmente, zusätzlich mit einer Deckschicht zu versehen. Zweckmäßigerweise wird man hierfür in bekannter Weise Schichten farbloser Oxide wählen, wie z.B. $TiO_2$, $ZrO_2$, $Al_2O_3$, $SbO_2$, ZnO, $SiO_2$, MgO, CaO, $Ce_2O_3$ oder $SnO_2$, die jeweils allein oder als Gemisch aufgebracht werden können. Für die Hydrophobierung der Oberfläche zur Anpassung an bestimmte Anwendungssysteme empfiehlt es sich, die erfindungsgemäßen Farbpigmente mit einem Silan oder oberflächenaktive Hilfsmitteln, wie z.B. Magnesiumstearat, zu behandeln. Eine solche Deckschicht kann nach den üblichen Methoden auf bereits getrocknete Pigmente aufgebracht werden oder auch noch einfacher vor der Abtrennung der Pigmente aus der Fällösung. Im allgemeinen ist die Deckschicht dünner als die Eisenoxid- bzw. Eisenoxidhydrat-Schicht.

Die erfindungsgemäßen glanzlosen Farbpigmente zeichnen sich zum einen durch eine hohe Farbintensität und ein hohes Deckvermögen und zum anderen durch eine überaus hohe Reproduzierbarkeit des Farbtons aus, was bei konventionellen Absorptionspigmenten häufig nicht der Fall ist. Daneben zeigen sie interessante Farbtöne in anwendungstechnischen Systemen. Weiterhin zeichnen sie sich durch ein sehr gutes skin-feeling und vorteilhafte Werte für die Ölabsorption aus. Aufgrund dieser Eigenschaften sind die Pigmente insbesondere geeignet für wäßrige und nicht wäßrige kosmetische Formulierungen, aber auch für kosmetische Feststoffzubereitungen, wie z.B. Puder, Salben, Emulsionen, Fettstiften und anderen Mitteln und können in Konzentrationen von in der Regel 0,01 bis 100 Gew.% eingesetzt werden. Die Farbpigmente weisen darüberhinaus eine gute Dispergierbarkeit und Redispergierbarkeit auf und werden daher vorzugsweise in wäßrigen oder nicht wäßrigen Beschichtungssystemen aus den Bereichen Lacke, Farben und Druckfarben verwendet. Weiterhin eignen sich die erfindungsgemäßen Pigmente zur Pigmentierung von Kunststoffen.

Gegenstand der Erfindung sind somit auch Formulierungen, die die erfindungsgemäßen Farbpigmente enthalten.

Kunststoffe enthaltend die erfindungsgemäßen Pigmente sind weiterhin geeignet für die Lasermarkierung. Durch den Zusatz der Farbpigmente in Konzentrationen von 0,01 bis 20 Gew.%, vorzugsweise 0,5 bis 15 Gew.%, insbesondere 0,8 bis 5 Gew.%, bezogen auf das Kunststoffsystem werden bei den Lasermarkierungen kontrastreiche und kantenscharfe Markierungen, die wisch- und kratzfest sind, erhalten. Die Verwendung dieses pigmentierten Kunststoffes kann auf allen Gebieten erfolgen, wo bisher übliche Druckverfahren zur Beschriftung von Kunststoffen eingesetzt werden,

Die nachfolgenden Beispiele sollen die Erfindung erläutern, ohne sie zu begrenzen:

Beispiel 1

100 g trocken gemahlener Glimmer mit einer Teilchengröße von < 12 μm werden in 2 l Wasser suspendiert. Mit 10%iger Salzsäure wird der pH-Wert auf 7,5 eingestellt. Bei 20 °C wird zu dieser Pigmentsuspension eine wäßrige $FeSO_4$-Lösung (120 g $FeSO_4 \cdot 7\,H_2O$ in 420 ml Wasser) zugetropft. Gleichzeitig wird Sauerstoff mit einer Rate von 30 l/min durch die Suspension geleitet. Während der Reaktion wird der pH-Wert mit 10%iger $Na_2CO_3$-Lösung auf pH = 7-8 konstant gehalten. Nach beendeter Zugabe der $FeSO_4$-Lösung wird noch 15 Minuten gerührt. Zuletzt wird mit Wasser sulfatfrei gewaschen, das Produkt bei 110 °C getrocknet und auf 100 μm gesiebt. Das fertige Pigment enthält 25,6 Gew.% FeO(OH) und zeichnet sich durch seine intensive gelb-orange Farbe aus.

Beispiel 2

Das Produkt aus Beispiel 1 wird 0,5 h bei 850 °C geglüht. Man erhält ein Orangepigment mit hoher Farbreinheit.

Vergleichsbeispiel 1

Der in Beispiel 1 verwendete trocken gemahlene Glimmer wird durch plättchenförmigen Glimmer der Teilchengröße < 15 μm ersetzt. Die Beschichtung mit FeO(OH) erfolgt analog Beispiel 1.

Vergleichsbeispiel 2

Das Produkt aus Vergleichsbeispiel 1 wird 0,5 h bei 850 °C geglüht.

Koloristische Bewertung

Die aus den Beispielen und den Vergleichsbeispielen erhaltenen Gelb- und Orangepigmente werden in eine Lackgrundlage (Nitrocelluloselack) eingearbeitet und auf Blechplatten aufgestrichen. Die Farb- und Glanzcharakteristik wird nach der Lab-Methode (DIN 5033 Hunter-LAB) gemessen. Aus den Helligkeitswerten (L-Werten) der Lackkarten - Meßgeometrien 22,5°/22,5° und 45°/0° - wird nach folgender Formel das reflektierte Licht (Glanz) aus unterschiedlich zurückgelegten Wegen erfaßt:

$$\text{Glanzzahl} = \frac{L_{22,5°/22,5°} - L_{45°/0°}}{L_{22,5°/22,5°}} \times 100$$

| Produkt | Glanzzahl |
|---|---|
| Beispiel 1 | 30,6 |
| Vergleichsbeispiel 1 | 50,6 |
| Beispiel 2 | 34,4 |
| Vergleichsbeispiel 2 | 42,8 |

Aus der Tabelle geht hervor, daß die aus den Beispielen 1 und 2 erhaltenen Produkte auf Basis von trocken gemahlenen Glimmer einen wesentlich geringeren Glanz zeigen als Pigmente auf Basis von plättchenförmigen Glimmer.

Patentansprüche

1. Nicht glänzende eisenoxidhaltige Farbpigmente auf Basis von trocken gemahlenen anorganischen plättchenförmigen Trägern der Teilchengröße < 25 μm, **dadurch gekennzeichnet, daß** glanzlose Basissubstrate mit unregelmäßiger Teilchenform mit gelbem FeO(OH) und/oder $Fe_2O_3$ beschichtet werden.

2. Farbpigmente nach Anspruch 1, **dadurch gekennzeichnet, daß** die eisenoxidhaltige Schicht einheitlich aus $Fe_2O_3$ besteht, das durch Entwässern von FeO(OH) bei Temperaturen von 400-1000 °C entstanden ist.

3. Farbpigmente nach Anspruch 1 oder 2, **dadurch gekennzeichnet, daß** der Eisenoxid- oder Eisenhydroxid-Gehalt 5 bis 70 Gew.% bezogen auf das Gesamtpigment beträgt.

4. Farbpigmente nach einem der Ansprüche 1 bis 3, **dadurch gekennzeichnet, daß** der anorganische plättchenförmige Träger Glimmer ist.

5. Farbpigmente nach einem der Ansprüche 1 bis 4, **dadurch gekennzeichnet, daß** sie zusätzlich eine Deckschicht aus ein oder mehreren farblosen Metalloxiden, Silanverbindungen oder Stearaten oder Kombinationen dieser Verbindungen enthalten.

6. Verfahren zur Herstellung von Farbpigmenten nach Anspruch 1, **dadurch gekennzeichnet, daß** man eine wäßrige Suspension des trocken gemahlenen anorganischen plättchenförmigen Trägers vorlegt, bei Temperaturen von 5-40 °C und einem pH-Wert von 6-9 mit einer wäßrigen Eisen(II)salzlösung versetzt und durch die Reaktionslösung Luft und/oder Sauerstoff leitet, danach das beschichtete Pigment abtrennt, wäscht, trocknet und gegebenenfalls bei Temperaturen von 400-1000 °C glüht.

7. Verwendung der Farbpigmente nach Anspruch 1 in Lacken, Farben, Kunststoffen und in kosmetischen Formulierungen.

8. Formulierungen enthaltend Farbpigmente nach Anspruch 1.

9. Lasermarkierbare Kunststoffe enthaltend Farbpigmente nach Anspruch 1.

## Claims

1. Non-lustrous iron oxide-containing colour pigments based on dry-milled inorganic plateletlike substrates having a particle size of < 25 μm, **characterized in that** the lustreless basic substrates of irregular particle morphology are coated with yellow FeO(OH) and/or $Fe_2O_3$.

2. Colour pigments according to Claim 1, **characterized in that** the iron oxide-containing layer consists uniformly of $Fe_2O_3$ which has been prepared by dehydrating FeO(OH) at temperatures of 400 - 1000°C.

3. Colour pigments according to Claim 1 or 2, **characterized in that** the content of iron oxide or iron hydroxide is from 5 to 70% by weight, based on the overall pigment.

4. Colour pigments according to one of Claims 1 to 3, **characterized in that** the inorganic plateletlike substrate is mica.

5. Colour pigments according to one of Claims 1 to 4, **characterized in that** they additionally contain a covering layer comprising one or more colourless metal oxides, silane compounds or stearates or combinations of these compounds.

6. Process for the preparation of colour pigments according to Claim 1, **characterized in that** an aqueous suspension of the dry-milled inorganic plateletlike substrate is used as initial charge, an aqueous iron(II) salt solution is added at temperatures of 5 - 40°C and at a pH of 6 - 9, and air and/or oxygen is passed through the reaction solution, after which the coated pigment is isolated, washed, dried and, if desired, calcined at temperatures of 400 - 1000°C.

7. Use of the colour pigments according to Claim 1 in coatings, inks, plastics and in cosmetic formulations.

8. Formulations comprising colour pigments according to Claim 1.

9. Laser-markable plastics comprising colour pigments according to Claim 1.

## Revendications

1. Pigments colorés non brillants contenant de l'oxyde de fer, à base de supports inorganiques lamellaires broyés à sec ayant une taille de particules inférieure à 25μm, **caractérisés en ce que** des substrats de base mats sont revêtus avec du FeO(OH) jaune et/ou du $Fe_2O_3$ avec une forme de particules irrégulière.

2. Pigments colorés selon la revendication 1, **caractérisés en ce que** la couche d'oxyde de fer se compose uniformément de $Fe_2O_3$ obtenu par déshydratation de FeO(OH) à des températures de 400 à 1.000°C.

3. Pigments colorés selon la revendication 1 ou la revendication 2, **caractérisés en ce que** la teneur en oxyde de fer ou en hydroxyde de fer est de 5 à 70% en poids, ramenée au poids de pigment total.

4. Pigments colorés selon l'une des revendications 1 à 3, **caractérisés en ce que** le support inorganique lamellaire est le mica.

5. Pigments colorés selon l'une des revendications 1 à 4, **caractérisés en ce qu'**ils contiennent en plus une couche de recouvrement faite d'un ou plusieurs oxydes métalliques, composés de silane ou stéarates incolores ou de combinaisons de ces composés.

6. Procédé de fabrication de pigments colorés selon la revendication 1, **caractérisé en ce que** l'on charge une suspension aqueuse du support inorganique lamellaire broyé à sec, on la mélange à des températures de 5 à 40°C et à un pH de 6 à 9 avec une solution aqueuse de sel de fer (II) et on fait passer de l'air et/ou de l'oxygène dans la solution réactionnelle, après quoi on sépare le pigment revêtu, on le lave, on le sèche et le cas échéant on le calcine à des températures de 400 à 1.000°C.

7. Utilisation des pigments colorés selon la revendication 1 dans des peintures, des colorants, des matières plastiques et dans des formulations cosmétiques.

8. Formulations contenant des pigments colorés selon la revendication 1.

9. Matières plastiques pouvant être marquées au laser, contenant des pigments colorés selon la revendication 1.